# FASCICULE DE BREVET EUROPEEN

(11) **EP 0 580 470 B1**
(45) Date de publication et mention de la délivrance du brevet: **19.05.1999**
(21) Numéro de dépôt: 93401699.9
(22) Date de dépôt: 30.06.1993
(51) Int. Cl.: A61F 5/453

(54) **Applicateur pour un cathéter externe de collecte urinaire, et dispositif de collecte urinaire comportant ledit applicateur**
Anlegevorrichtung für einen externen Harnkatheter und Harnkatheter der eine solche Anlegevorrichtung enthält
Applicator for an external urinary catheter and urinary catheter incorporating such applicator

(30) Priorité: 20.07.1992 FR 9208927
(43) Date de publication de la demande: 26.01.1994
(73) Titulaire: PORGES, 92350 Le Plessis-Robinson (FR)
(72) Inventeur: Bailly, Pierre, F-92120 Montrouge (FR)
(74) Mandataire: Bonnetat, Christian

(56) Documents cités:
- EP-A- 0 284 224
- WO-A-88/02624
- US-A- 4 589 874

## Description

La présente invention concerne un applicateur pour un cathéter externe de collecte urinaire pour individus de sexe masculin, et un dispositif de collecte urinaire comportant ledit applicateur.

On connaît des dispositifs de collecte urinaire comportant essentiellement un cathéter externe comprenant un manchon souple destiné à être appliqué de façon étanche sur le pénis d'un patient, lequel manchon est raccordé à une partie de réception du gland, sensiblement en forme de calotte sphérique, elle-même raccordée à un tube d'évacuation de l'urine, ledit manchon étant enroulé sur lui-même préalablement à son application. Une difficulté consiste en la mise en place d'un tel cathéter du fait du manque de rigidité du pénis généralement constatée chez le patient au moment de l'application du cathéter. Pour mettre en place ce dernier, il est donc nécessaire d'utiliser un applicateur. Un ensemble cathéter-applicateur est décrit, notamment, dans le brevet US-A-4 589 874. Dans ce cas, l'applicateur est constitué d'un corps tubulaire destiné à entourer partiellement la partie de réception du gland, et présente un bourrelet derrière lequel le manchon est maintenu en position enroulée, en permettant d'éviter un déroulement involontaire dudit manchon. Par ailleurs, le corps présente des évidements qui, lors de la mise en place du cathéter, servent d'ouvertures pour les doigts de l'opérateur pour saisir la partie de réception du gland et l'extrémité de ce dernier, alors que, de l'autre main, l'opérateur déroule le manchon. Cependant, dans ces circonstances, on constate souvent une rétraction du pénis telle qu'elle empêche la mise en place d'un tel cathéter malgré l'emploi d'un applicateur, notamment comme celui décrit dans le brevet US-A-4 589 874.

La présente invention a pour but d'éviter cet inconvénient, et concerne un applicateur pour un cathéter externe de collecte urinaire, adapté pour maintenir, de façon positive, le pénis, et l'empêcher de se rétracter pendant la mise en place du cathéter.

A cet effet, l'applicateur pour cathéter externe de collecte urinaire, ledit cathéter comprenant un manchon souple destiné à être appliqué de façon étanche sur le pénis d'un patient, lequel manchon est raccordé à une partie de réception du gland, de forme tubulaire, elle-même raccordée à un tube d'évacuation de l'urine, ledit manchon étant enroulé sur lui-même préalablement à son application, est remarquable, selon l'invention, en ce que ledit applicateur comporte une bague de diamètre variable, présentant un diamètre initial tel qu'elle puisse être enfilée sur ladite partie de réception du gland, ledit diamètre initial pouvant être réduit de façon commandable de sorte que ladite bague puisse venir s'accrocher derrière le gland et maintenir celui-ci pendant le déroulement du manchon souple sur le pénis, et ladite bague pouvant reprendre spontanément son diamètre initial de façon à pouvoir retirer ledit applicateur dudit cathéter.

Ainsi, en saisissant la bague de façon à la faire venir s'accrocher derrière le gland, l'opérateur peut tirer la verge de façon à provoquer son extension pour de ce fait faciliter le déroulement du manchon sur celle-ci.

Avantageusement, ladite bague comporte une partie périphérique, s'étendant sur la majeure partie de la circonférence de la bague, et deux segments périphériques adjacents, délimités, chacun, par des lignes de pliage de moindre épaisseur, de sorte que, en pressant l'un vers l'autre les deux dits segments, la bague est réduite à une bague sensiblement définie par ladite partie périphérique.

De préférence, les deux dits segments s'étendent sur 120° symétriquement par rapport à leur ligne de pliage commune.

Pour faciliter la préhension de l'applicateur par l'opérateur et permettre aisément la réduction de diamètre de la bague, lesdits deux segments peuvent être formés en une seule pièce avec une partie de préhension en forme d'auge, s'étendant transversalement à ladite bague dans le prolongement des deux dits segments, et elle-même séparée en deux portions par le prolongement de la ligne de pliage commune aux deux dits segments.

Cependant, selon une variante de réalisation, l'applicateur peut comprendre une partie de préhension en forme d'auge, s'étendant transversalement à ladite bague, séparée en deux portions par une ligne de pliage centrale, et solidaire, à ses extrémités, de ladite bague au voisinage des lignes de pliage reliant les deux dits segments à ladite partie périphérique. Cela permet, avant l'application, de maintenir le manchon enroulé sur lui-même sur ladite bague de façon non tendue, en rentrant les deux segments vers l'intérieur de la bague, et d'éviter ainsi que le manchon, une fois déroulé, ne forme des plis.

Avantageusement, ladite bague comporte une gorge périphérique de réception dudit manchon en position enroulée sur lui-même.

Les figures du dessin annexé feront bien comprendre comment l'invention peut être réalisée. Sur ces figures, des références identiques désignent des éléments semblables.
La figure 1 est une vue en perspective d'un premier exemple de réalisation d'un applicateur pour un cathéter externe de collecte urinaire selon l'invention.
La figure 2 est une vue de face du cathéter de la figure 1.
La figure 3 est une vue de côté du cathéter de la figure 1.
Les figures 4a et 4b illustrent, en vue de face et en vue de côté, une première étape de la mise en place d'un cathéter externe de collecte urinaire sur le pénis d'un patient, en utilisant l'applicateur des figures 1 à 3.
Les figures 5a et 5b illustrent, en vue de face et en vue de côté, une deuxième étape de cette mise en place.
Les figures 6 et 7 montrent les dernières étapes de cette mise en place.
La figure 8 est une vue en perspective d'un second exemple de réalisation d'un applicateur pour un cathéter externe de collecte urinaire selon l'invention.
La figure 9 est une vue de face du cathéter de la figure 8.
La figure 10 est une vue de côté du cathéter de la figure 8.
Les figures 11a et 11b illustrent, en vue de face et en vue de côté, le cathéter externe de collecte urinaire et son applicateur, réalisé conformément au second exemple montré sur les figures 8 à 10, avant application.
Les figures 12a et 12b illustrent, en vue de face et en vue de côté, une première étape de la mise en place du cathéter externe de collecte urinaire, selon les figures 11a et 11b, sur le pénis d'un patient, en utilisant l'applicateur des figures 8 à 10.
Les figures 13a et 13b illustrent, en vue de face et en vue de côté, une deuxième étape de cette mise en place.
Les figures 14 et 15 montrent les dernières étapes de cette mise en place.

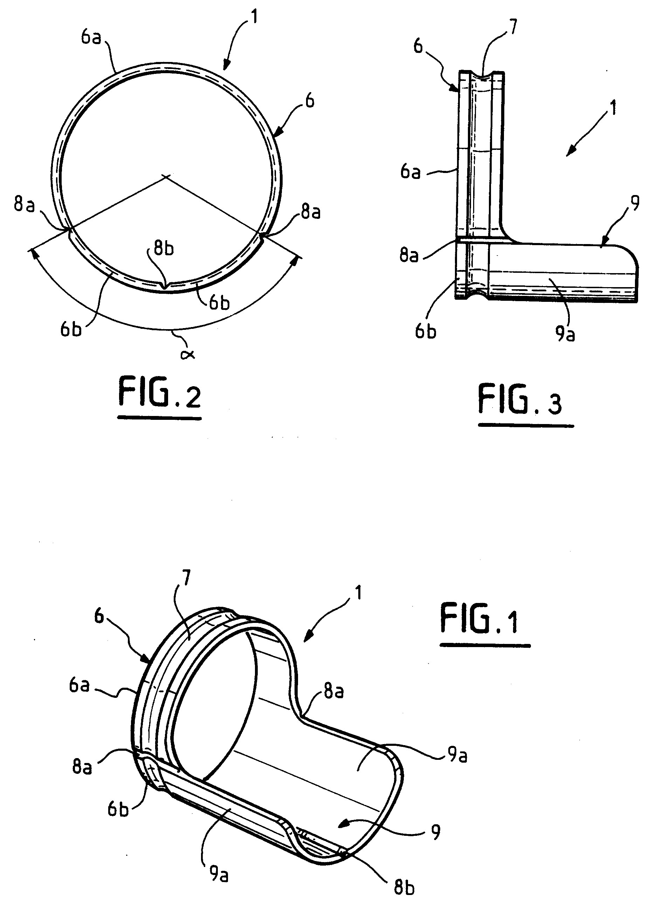

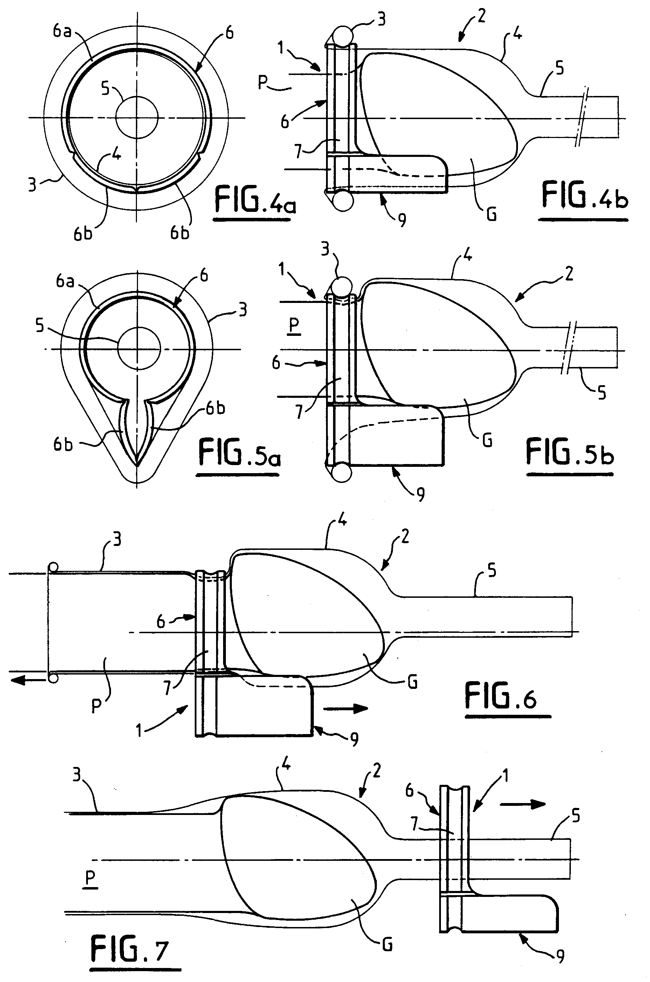

Sur les figures 1 à 3, on a représenté un premier exemple de réalisation d'un applicateur 1 selon l'invention pour un cathéter externe 2 de collecte urinaire, montré sur les figures 4a, 4b ; 5a, 5b ; 6 et 7. Ce dernier comporte, de façon classique, un manchon souple 3, destiné à être appliqué sur le pénis d'un patient, qui est raccordé à une partie de réception 4 du gland, de forme cylindrique, elle-même raccordée à un tube 5 d'évacuation de l'urine (pouvant être relié à des moyens de collecte de l'urine non montrés).

L'applicateur 1, quant à lui, comporte une bague 6 de diamètre variable, pourvue d'une gorge périphérique 7 de réception du manchon 3 en position enroulée sur lui-même. La bague 6 présente une partie périphérique 6a, s'étendant sur la majeure partie de la circonférence de la bague, et deux segments périphériques adjacents 6b, délimités, chacun, par des lignes de pliage de moindre épaisseur 8a (les reliant à la partie périphérique 6a) et 8b (les reliant l'un à l'autre). Dans cet exemple de réalisation, les deux segments 6b s'étendent sur un angle α de 120° symétriquement par rapport à leur ligne de pliage commune 8b. Par ailleurs, les deux segments 6b sont formés en une seule pièce avec une partie de préhension 9 en forme d'auge, s'étendant transversalement à la bague 6 (c'est-à-dire sensiblement coaxialement à la partie de réception 4 du gland) dans le prolongement des segments 6b, et elle-même séparée en deux portions 9a par le prolongement de la ligne de pliage 8b commune aux segments 6b.

Les figures 4a, 4b ; 5a, 5b ; 6 et 7 montrent la mise en place du cathéter externe de collecte urinaire 2 sur le pénis P d'un patient à l'aide de l'applicateur 1 des figures 1 à 3. L'applicateur 1 étant en place sur le cathéter 2 et le manchon 3 de ce dernier étant disposé, à l'état enroulé, dans la gorge 7 de la bague 6, l'ensemble applicateur-cathéter est enfilé sur le gland G du pénis P (la bague 6 doit donc présenter un diamètre initial légèrement plus grand que celui du gland G), la partie de réception 4 entourant complètement le gland et la bague 6 de l'applicateur se trouvant au-delà du gland (la partie de préhension 9 de l'applicateur 1 est alors en vis-à-vis du dessous du gland G en s'étendant sur une portion de la partie de réception 4). Cette étape initiale est montrée sur la figure 4b. En pressant l'une vers l'autre les deux portions 9a de la partie de préhension 9, c'est-à-dire également les segments 6b de la bague 6, cette dernière est réduite à une bague sensiblement définie par la partie périphérique 6a (figures 5a et 5b), de sorte que la bague peut venir s'accrocher derrière le gland G, et l'opérateur peut ainsi tirer sur la verge du patient, en évitant la rétraction de cette dernière, tout en déroulant le manchon 3 sur le pénis P (figure 6). Lorsque le manchon 3 est totalement déroulé, le cathéter 2 est en place, et l'opérateur, en relâchant la pression sur la partie de préhension 9, permet à la bague de reprendre spontanément son diamètre initial (figures 4a et 4b) de façon à pouvoir retirer l'applicateur 1 du cathéter 2 (figure 7).

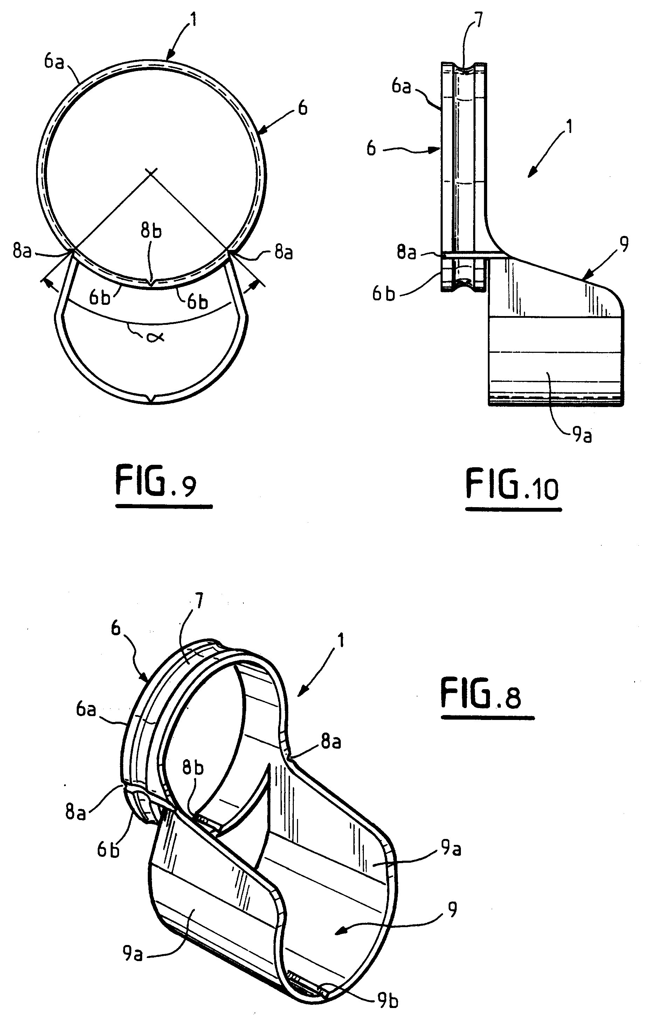

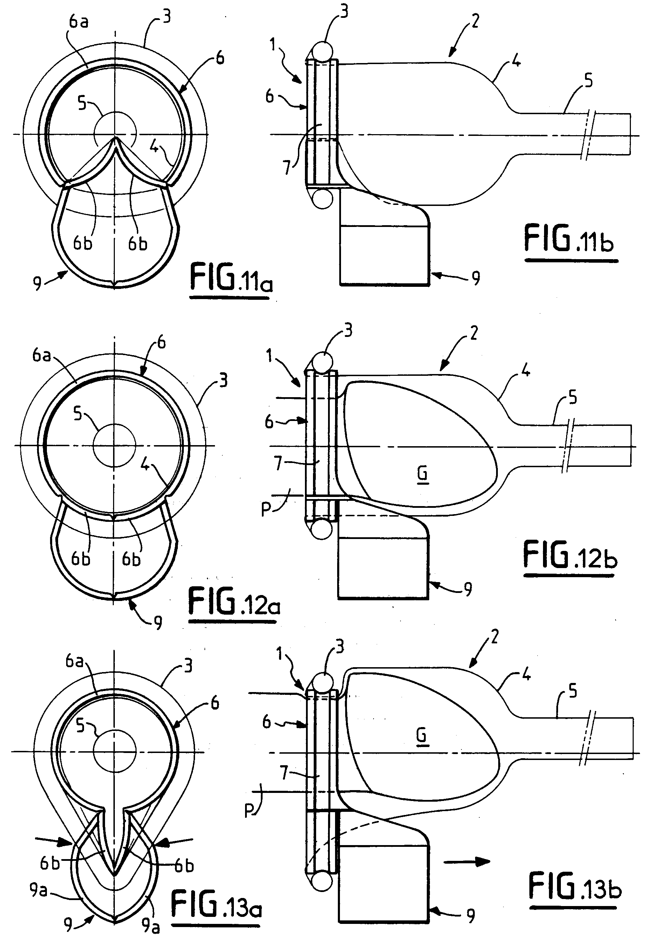

Sur les figures 8 à 10, on a représenté un second exemple de réalisation d'un applicateur 1 selon l'invention pour un cathéter externe 2 de collecte urinaire montré sur les figures 11a, 11b ; 12a, 12b ; 13a, 13b ; 14 et 15, ce dernier étant du même type que celui précédemment décrit et ces éléments étant désignés par les mêmes références numériques.

Par ailleurs, cet exemple de réalisation de l'applicateur 1 comporte la plupart des éléments de l'applicateur 1 des figures 1 à 3, désignés sur les figures 8 à 10 par les mêmes références numériques. Toutefois, dans ce cas, la partie de préhension 9 en forme d'auge, s'étendant transversalement à la bague 6, et séparée en deux portions 9a par une ligne de pliage centrale 9b, n'est plus formée en une seule pièce avec les segments 6b (constituant avec la partie périphérique 6a la bague 6), mais est solidaire, à ses extrémités, de la bague 6 au voisinage des lignes de pliage 8a reliant les segments 6b à la partie périphérique 6a de la bague 6. Comme on le voit sur les figures 8 à 10, la partie de préhension 9 pend donc de la bague 6 en "libérant" les segments 6b de cette dernière.

Les figures 11a et 11b montrent l'intérêt de cette configuration. En effet, comme on le voit, celle-ci permet, avant l'application, de maintenir le manchon 3 enroulé sur lui-même dans la gorge 7 de la bague 6 de façon non tendue, en rentrant les deux segments 6b vers l'intérieur de la bague 6, et d'éviter ainsi que le manchon 3, une fois déroulé, ne forme des plis.

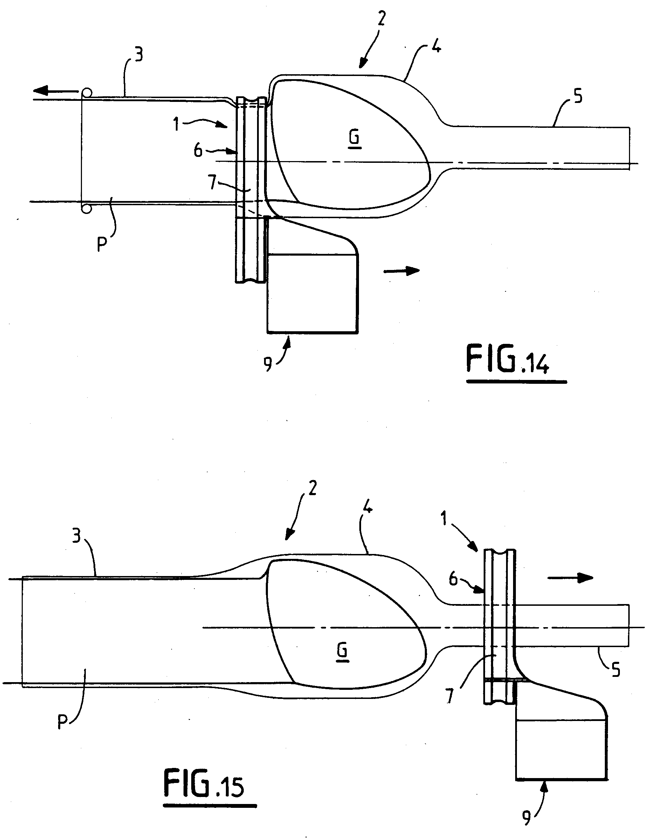

Par ailleurs, la mise en place du cathéter externe de collecte urinaire 2 sur le pénis P d'un patient à l'aide de l'applicateur 1 des figures 8 à 10 est illustrée sur les figures 12a, 12b ; 13a, 13b ; 14 et 15. L'applicateur 1 étant en place sur le cathéter 2 et le manchon 3 de ce dernier étant disposé, à l'état enroulé, dans la gorge 7 de la bague 6 (les segments 6b étant alors, bien entendu, ramenés dans la position où ils sont dans le prolongement de la partie périphérique 6a en tendant le manchon 3, comme on le voit sur la figure 12a), l'ensemble applicateur-cathéter est enfilé sur le gland G du pénis P, la partie de réception 4 entourant complètement le gland et la bague 6 de l'applicateur se trouvant au-delà du gland (la partie de préhension 9 de l'applicateur 1 est alors au-dessous du gland G en s'étendant sur une portion de la partie de réception 4). Cette étape initiale est montrée sur la figure 12b. En pressant l'une vers l'autre les deux portions 9a de la partie de préhension 9, c'est-à-dire également les segments 6b de la bague 6 du fait de la solidarisation entre la partie de préhension 9 et les segments 6b au niveau des lignes de pliage 8a, la bague est réduite à une bague sensiblement définie par la partie périphérique 6a (figures 13a et 13b), de sorte que la bague peut venir s'accrocher derrière le gland G. L'opérateur peut ainsi tirer sur la verge du patient, en évitant la rétraction de cette dernière, tout en déroulant le manchon 3 sur le pénis P (figure 14). Lorsque le manchon 3 est totalement déroulé, le cathéter 2 est en place, et l'opérateur, en relâchant la pression sur la partie de préhension 9, permet à la bague de reprendre spontanément son diamètre initial (figures 12a et 12b) de façon à pouvoir retirer l'applicateur 1 du cathéter 2 (figure 15).

## Revendications

1. Applicateur pour cathéter externe de collecte urinaire, ledit cathéter (2) comprenant un manchon souple (3) destiné à être appliqué de façon étanche sur le pénis d'un patient, lequel manchon (3) est raccordé à une partie (4) de réception du gland, de forme tubulaire, elle-même raccordée à un tube (5) d'évacuation de l'urine, ledit manchon (3) étant enroulé sur lui-même préalablement à son application,
caractérisé en ce que ledit applicateur (1) comporte une bague (6) de diamètre variable, présentant un diamètre initial tel qu'elle puisse être enfilée sur ladite partie (4) de réception du gland, ledit diamètre initial pouvant être réduit de façon commandable de sorte que ladite bague (6) puisse venir s'accrocher derrière le gland et maintenir celui-ci pendant le déroulement du manchon souple (3) sur le pénis, et ladite bague (6) pouvant reprendre spontanément son diamètre initial de façon à pouvoir retirer ledit applicateur (1) dudit cathéter (2).

2. Applicateur selon la revendication 1,
caractérisé en ce que ladite bague (6) comporte une partie périphérique (6a), s'étendant sur la majeure partie de la circonférence de la bague, et deux segments périphériques adjacents (6b), délimités, chacun, par des lignes de pliage (8a,8b) de moindre épaisseur, de sorte que, en pressant l'un vers l'autre les deux dits segments (6b), la bague (6) est réduite à une bague sensiblement définie par ladite partie périphérique (6a).

3. Applicateur selon la revendication 2,
caractérisé en ce que les deux dits segments (6b) s'étendent sur 120° symétriquement par rapport à leur ligne de pliage commune (8b).

4. Applicateur selon la revendication 2 ou la revendication 3,
caractérisé en ce que lesdits deux segments (6b) sont formés en une seule pièce avec une partie de préhension (9) en forme d'auge, s'étendant transversalement à ladite bague (6) dans le prolongement des deux dits segments (6b), et elle-même séparée en deux portions (9a) par le prolongement de la ligne de pliage (8b) commune aux deux dits segments (6b).

5. Applicateur selon la revendication 2 ou la revendication 3,
caractérisé en ce qu'il comprend une partie de préhension (9) en forme d'auge, s'étendant transversalement à ladite bague (6), séparée en deux portions (9a) par une ligne de pliage centrale (9b), et solidaire, à ses extrémités, de ladite bague (6) au voisinage des lignes de pliage (8a) reliant les deux dits segments (6b) à ladite partie périphérique (6a).

6. Applicateur selon l'une quelconque des revendications 1 à 5,
caractérisé en ce que ladite bague (6) comporte une gorge périphérique (7) de réception dudit manchon (3) en position enroulée sur lui-même.

7. Dispositif de collecte urinaire, comprenant un cathéter externe (2) comportant un manchon souple (3) destiné à être appliqué de façon étanche sur le pénis d'un patient, lequel manchon (3) est raccordé à une partie de réception du gland (4), de forme tubulaire, elle-même raccordée à un tube (5) d'évacuation de l'urine, ledit manchon (3) étant enroulé sur lui-même préalablement à son application,
caractérisé en ce qu'il comprend un applicateur (1) tel que défini dans l'une quelconque des revendications 1 à 6.

## Claims

1. An applicator for an external urinary collection catheter, said catheter (2) comprising a flexible sheath (3) intended to be applied in a leaktight manner onto the penis of a patient, which sheath (3) is connected to a part (4), for receiving the glans, of tubular shape, itself connected to a tube (5) for removing urine, said sheath (3) being rolled up prior to its application, characterized in that said applicator (1) comprises a ring (6) of variable diameter, having an initial diameter such that it can be slipped over said part (4) for receiving the glans, said initial diameter being capable of being reduced, in a controlled manner, such that said ring (6) can be caught behind the glans and hold the latter during the unrolling of the flexible sheath (3) over the penis, and said ring (6) being capable of resuming its initial diameter spontaneously, so that the said applicator (1) can be removed from the said catheter (2).

2. The applicator as claimed in claim 1, characterized in that said ring (6) comprises a peripheral part (6a), extending over most of the circumference of the ring, and two adjacent peripheral segments (6b), each delimited by folding lines (8a, 8b) of lesser thickness, such that, by pressing the two said segments (6b) towards each other, the ring (6) is reduced to a ring substantially defined by said peripheral part (6a).

3. The applicator as claimed in claim 2, characterized in that the two said segments (6b) extend over 120° symmetrically with respect to their common folding line (8b).

4. The applicator as claimed in claim 2 or claim 3, characterized in that said two segments (6b) are formed in a single piece with a trough-shaped gripping part (9), extending transversely to said ring (6) in the extension of the two said segments (6b), and itself separated into two portions (9a) by the extension of the folding line (8b) common to the two said segments (6b).

5. The applicator as claimed in claim 2 or claim 3, characterized in that it comprises a trough-shaped gripping part (9), extending transversely to said ring (6), separated into two portions (9a) by a central folding line (9b), and integral, at its ends, with said ring (6), in the vicinity of the folding lines (8a) joining the two said segments (6b) to said peripheral part (6a).

6. The applicator as claimed in any one of claims 1 to 5, characterized in that said ring (6) comprises a peripheral groove (7) for receiving said sheath (3) in the rolled-up position.

7. A urinary collection device, which comprises an external catheter (2), comprising a flexible sheath (3) intended to be applied in a leaktight manner onto the penis of a patient, which sheath (3) is connected to a part for receiving the glans (4), of tubular shape, itself connected to a tube (5) for removing urine, said sheath (3) being rolled up prior to its application, characterized in that it comprises an applicator (1) as defined in any one of claims 1 to 6.

## Patentansprüche

1. Anlegevorrichtung für einen externen Harnkatheter, wobei der Katheter (2) eine elastische Manschette (3) umfaßt, die dazu bestimmt ist, in dichter Weise am Penis eines Patienten angelegt zu werden, wobei die Manschette (3) mit einem röhrenförmigen Teil (4) zur Aufnahme der Eichel verbunden ist, der seinerseits mit einer Röhre (5) zum Ablassen des Urins verbunden ist und wobei die Manschette (3) vor ihrem Anlegen auf sich selbst aufgerollt ist,
dadurch gekennzeichnet, daß die Anlegevorrichtung (1) einen Ring (6) mit variablem Durchmesser umfaßt, der einen solchen anfänglichen Durchmesser aufweist, daß er über den Teil (4) zur Aufnahme der Eichel gestreift werden kann, wobei der anfängliche Durchmesser in steuerbarer Weise verringert wird, so daß sich der Ring (6) hinter der Eichel festklammern und diese während des Entrollens der elastischen Manschette (3) über dem Penis halten kann, und wobei der Ring (6) seinen anfänglichen Durchmesser spontan wieder annehmen kann, um die Anlegevorrichtung (1) des Katheters (2) entfernen zu können.

2. Anlegevorrichtung nach Anspruch 1,
dadurch gekennzeichnet, daß der Ring (6) einen Umfangsteil (6a), der sich über den größten Teil des Umfangs des Rings erstreckt, und zwei angrenzende Umfangssegmente (6b) aufweist, die jeweils von Biegelinien (8a, 8b) mit geringerer Dicke begrenzt sind, so daß der Ring (6) unter Gegeneinanderdrücken der beiden Segmente (6b) zu einem Ring verringert wird, der im Wesentlichen durch den Umfangsteil (6a) gebildet ist.

3. Anlegevorrichtung nach Anspruch 2,
dadurch gekennzeichnet, daß sich die beiden Segmente (6b) symmetrisch in bezug auf ihre gemeinsame Biegelinie (8b) über 120° erstrecken.

4. Anlegevorrichtung nach Anspruch 2 oder Anspruch 3,
dadurch gekennzeichnet, daß die beiden Segmente (6b) in einem einzigen Stück mit einem trogförmigen Griffteil (9) ausgebildet sind, der sich quer zum Ring (6) in der Verlängerung der beiden Segmente (6b) erstreckt und selbst durch die Verlängerung der den beiden Segmenten (6b) gemeinsamen Biegelinie (8b) in zwei Abschnitte (9a) unterteilt ist.

5. Anlegevorrichtung nach Anspruch 2 oder Anspruch 3,
dadurch gekennzeichnet, daß sie einen trogförmigen Griffteil (9) umfaßt, der sich quer zum Ring (6) erstreckt, durch eine mittlere Biegelinie (9b) in zwei Abschnitte (9a) unterteilt und an seinen Enden in der Nähe der Biegelinien (8a), die die beiden Segmente (6b) mit dem Umfangsteil (6a) verbinden, mit dem Ring (6) fest verbunden ist.

6. Anlegevorrichtung nach einem der Ansprüche 1 bis 5,
dadurch gekennzeichnet, daß der Ring (6) eine Umfangsrille (7) zur Aufnahme der Manschette (3) in auf sich selbst aufgerollter Position aufweist.

7. Harnkatheter, der einen externen Katheter (2) umfaßt, der eine elastische Manschette (3) aufweist, die dazu bestimmt ist, in dichter Weise am Penis eines Patienten angelegt zu werden, wobei die Manschette (3) mit einem röhrenförmigen Teil (4) zur Aufnahme der Eichel verbunden ist, der wiederum mit einer Röhre (5) zum Ablassen des Urins verbunden ist, und wobei die Manschette (3) vor dem Anlegen auf sich selbst aufgerollt ist,
dadurch gekennzeichnet, daß er eine Anlegevorrichtung (1) wie in einem der Ansprüche 1 bis 6 definiert umfaßt.
